# EUROPEAN PATENT APPLICATION

(11) **EP 4 830 616 A1**
(43) Date of publication of application: **28.10.2026**
(21) Application number: 24907328.9
(22) Date of filing: 13.12.2024
(51) Int. Cl.: A61B 5/00, A61B 1/24

(54) **ORAL CAVITY STATE ANALYSIS SYSTEM, ORAL CAVITY STATE ANALYSIS PROGRAM, STORAGE MEDIUM FOR STORING ORAL CAVITY STATE ANALYSIS PROGRAM, AND ORAL CAVITY STATE ANALYSIS METHOD**

(30) Priority: 22.12.2023 JP 2023216772
(71) Applicant: SUNSTAR INC., Takatsuki-shi, Osaka 569-1195 (JP)
(72) Inventor: HASEGAWA, Taira, Takatsuki-shi, Osaka 569-1195 (JP); KANATA, Takeshi, Takatsuki-shi, Osaka 569-1195 (JP); INOUE, Masaharu, Takatsuki-shi, Osaka 569-1195 (JP)
(74) Representative: Vossius & Partner Patentanwälte Rechtsanwälte mbB
(86) International application number: PCT/JP2024/044263
(87) International publication number: WO 2025/134943

(57) **Abstract**

[Purpose]

The present invention provides an oral cavity condition analysis system and an oral cavity condition analysis program, which enable easy obtaining of oral cavity images suitable for analyzing oral cavity conditions, and analyze the oral cavity conditions.

[Means to achieve the purpose]

The present invention includes: an image acceptance unit 251 that continuously accepts a face image V of a user U, which has been captured by the capturing unit 21 of a user terminal 2, as an input from a capturing unit 21; an image detection unit 252 that detects a mouth image F from the face image V; a tooth detection unit 253 that detects a tooth area TA from the mouth image F; a gum detection unit 254 that detects a gum area GA from the mouth image F; a determination unit 256 that determines whether at least one of the tooth area TA and the gum area GA which are included in the mouth image F; an image obtaining unit 257 that obtains one or more mouth images F that have been determined by the determination unit 256 to satisfy the condition; and an analysis unit 258 that analyzes the oral cavity condition based on the mouth image F obtained by the image obtaining unit 257.

## Description

### TECHNICAL FIELD

The present invention relates to an oral cavity condition analysis system for analyzing an oral cavity condition, an oral cavity condition analysis program, a storage medium storing the oral cavity condition analysis program, and a method of analyzing an oral cavity condition.

### BACKGROUND ART

In recent years, there have been proposed analysis systems that analyze oral cavity conditions based on oral cavity images captured by cameras in mobile terminals such as smartphones, with advances in technology (see Patent Literature (PTL) 1, for example).

### CITATIONS LIST

### PATENT LITERATURE

PTL 1: International Patent Application Publication No. WO2015/016377

### SUMMARY OF INVENTION

### TECHNICAL PROBLEMS

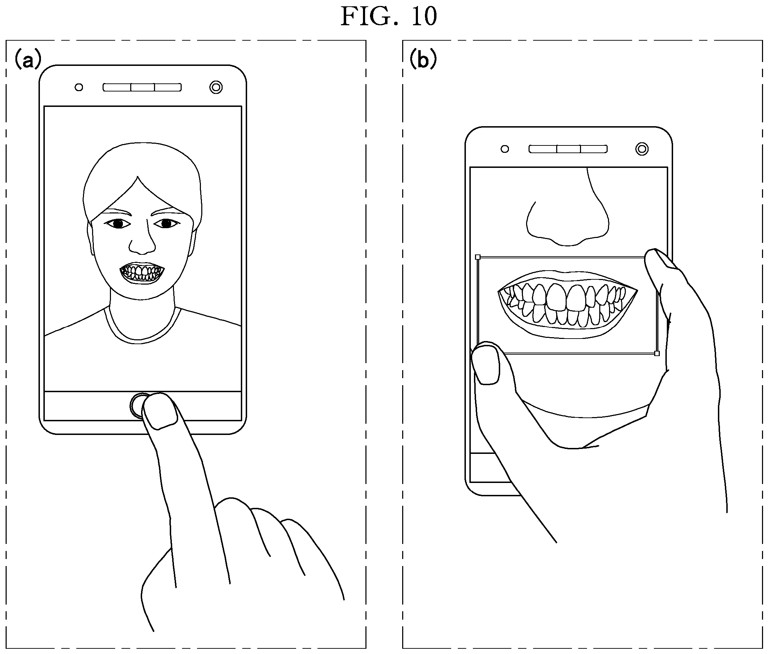

In such analysis systems, a user performed an operation of capturing a face image shown in FIG. 10A and an operation of extracting an oral cavity image shown in FIG. 10B from the face image for analysis. FIG. 10 includes schematic diagrams each illustrating the operations performed by a user in a conventional analysis system. Here, these face and oral cavity images are selected by the user, and thus these images do not necessarily include a portion or area suitable for analyzing an oral cavity condition. In addition, users had to repeatedly capture face images and extract oral cavity images from the captured face images until the user obtained an image deemed suitable for analyzing their oral cavity conditions, which lacked convenience.

A purpose of the present invention is to provide an oral cavity condition analysis system and an oral cavity condition analysis program, which enable automatic obtaining of oral cavity images suitable for analyzing oral cavity conditions.

### SOLUTIONS TO PROBLEMS

Specifically, the present invention encompasses the following inventions.
(1) An oral cavity condition analysis system including: an image acceptance unit that continuously accepts a face image of a user as an input from a capturing unit, the face image having been captured by the capturing unit provided in a user terminal; an image detection unit that detects an image containing a mouth area from the face image accepted by the image acceptance unit; a tooth detection unit that detects a tooth area from the image containing the mouth area; a gum detection unit that detects a gum area from the image containing the mouth area; a determination unit that determines whether at least one of the tooth area and the gum area which are included in the image containing the mouth area satisfies a predetermined condition necessary for analyzing an oral cavity condition; an image obtaining unit that obtains one or more images containing the mouth area, the images having been determined by the determination unit to satisfy the condition; and an analysis unit that analyzes the oral cavity condition based on the image containing the mouth area, the image having been obtained by the image obtaining unit.
   With this configuration, it is possible to automatically obtain oral cavity images suitable for analyzing oral cavity conditions without requiring repeated user input, thereby providing the convenient oral cavity condition analysis system with the usability.
(2) The oral cavity condition analysis system according to (1) including an exclusion target detection unit that detects an exclusion target other than the tooth area and the gum area from the image containing the mouth area.
   With this configuration, an area other than the tooth area and the gum area is detected as the exclusion target upon analyzing the oral cavity conditions, thereby defining the tooth and gum areas more clearly and improving accuracy of the analysis.
(3) The oral cavity condition analysis system according to (1), in which the determination unit records at least one of the tooth area and the gum area as time-series data, which vary for each image containing the mouth area, and determines whether the image containing the mouth area satisfies the predetermined condition necessary for analyzing the oral cavity condition based on the time-series data, the tooth area and the gum area.
   With this configuration, at least one of the tooth area and the gum area respectively detected by the tooth detection unit and the gum detection unit is recorded as the time-series data, thereby allowing the determination unit to accurately determine whether the predetermined conditions necessary for analyzing the oral cavity conditions are satisfied.
(4) The oral cavity condition analysis system according to (3), in which the user terminal includes a display screen on which the face image is displayed, and the display screen is provided with a guidance display unit that displays a guidance means for allowing the mouth area to be guided to a predetermined position.
   With this configuration, the mouth area of a user is guided, by the guidance means displayed on the guidance display unit, to a position where the image containing the mouth area, which satisfies the predetermined conditions necessary for analyzing the oral cavity conditions, is easily obtained on the display screen of the user terminal, thereby efficiently obtaining the image containing the mouth area.
(5) The oral cavity condition analysis system according to (3), in which the tooth detection unit extracts the tooth area from the image containing the mouth area using color space conversion and a predetermined threshold value, and the gum detection unit applies a differential filter to the image containing the mouth area, and detects the gum area using a flattening filter and a predetermined threshold value.
(6) An oral cavity condition analysis program that causes a computer to function as: an image acceptance unit that continuously accepts a face image of a user as an input from a capturing unit, the face image being captured by the capturing unit provided in a user terminal; an image detection unit that detects an image containing a mouth area from the face image accepted by the image acceptance unit; a tooth detection unit that detects a tooth area from the image containing the mouth area; a gum detection unit that detects a gum area from the image containing the mouth area; a determination unit that determines whether at least one of the tooth area and the gum area which are included in the image containing the mouth area satisfies a predetermined condition necessary for analyzing an oral cavity condition; an image obtaining unit that obtains one or more images containing the mouth area determined by the determination unit to satisfy the condition; and an analysis unit that analyzes the oral cavity condition based on the image containing the mouth area, the image having been obtained by the image obtaining unit.
(7) A computer readable recording medium that has an oral cavity condition analysis program recorded thereon for causing a computer to function as: an image acceptance unit that continuously accepts a face image of a user as an input from a capturing unit, the face image being captured by the capturing unit provided in a user terminal; an image detection unit that detects an image containing a mouth area from the face image accepted by the image acceptance unit; a tooth detection unit that detects a tooth area from the image containing the mouth area; a gum detection unit that detects a gum area from the image containing the mouth area; a determination unit that determines whether at least one of the tooth area and the gum area which are included in the image containing the mouth area satisfies a predetermined condition necessary for analyzing an oral cavity condition; an image obtaining unit that obtains one or more images containing the mouth area determined by the determination unit to satisfy the condition; and an analysis unit that analyzes the oral cavity condition based on the image containing the mouth area, the image having been obtained by the image obtaining unit.
(8) A method of analyzing an oral cavity condition, the method comprising: continuously accepting a face image of a user as an input from a capturing unit, the face image having been captured by the capturing unit provided in a user terminal; detecting an image containing a mouth area from the face image; detecting a tooth area and a gum area from the image containing the mouth area; determining whether at least one of the tooth area and the gum area which are included in the image containing the mouth area satisfies a predetermined condition necessary for analyzing an oral cavity condition; obtaining one or more images containing the mouth area, the images having been determined to satisfy the condition; and analyzing the oral cavity condition based on the image containing the mouth area, the image having been obtained.
   With this method, it is possible to automatically obtain oral cavity images suitable for analyzing the oral cavity conditions without requiring repeated user input, thereby providing the convenient method for analyzing oral cavity conditions.
(9) The method of analyzing an oral cavity condition according to (8), further including detecting an exclusion target other than the tooth area and the gum area from the image containing the mouth area.
   With this method, an area other than the tooth area and the gum area is detected as the exclusion target upon analyzing the oral cavity condition, thereby defining the tooth area and the gum area more clearly and improving accuracy of the analysis.
(10) The method of analyzing an oral cavity condition according to (8) further including: recording at least one of the tooth area and the gum area, which vary for each image including the mouth area, as time-series data; and determining whether the image containing the mouth area satisfies the predetermined condition necessary for analyzing the oral cavity condition based on the time-series data.

With this method, at least one of the tooth area and the gum area respectively detected by the tooth detection unit and the gum detection unit is recorded as the time-series data, thereby allowing the determination unit to accurately determine whether the predetermined conditions necessary for analyzing the oral cavity conditions are satisfied.

### ADVANTAGEOUS EFFECTS OF INVENTION

According to the present invention, it is possible to provide an oral cavity condition analysis system, an oral cavity condition analysis program, a storing medium storing the oral cavity analysis program, and an oral cavity analysis method, which enable automatic obtaining of oral cavity images suitable for analyzing oral cavity conditions.

### BRIEF DESCRIPTION OF DRAWINGS

[FIG. 1]

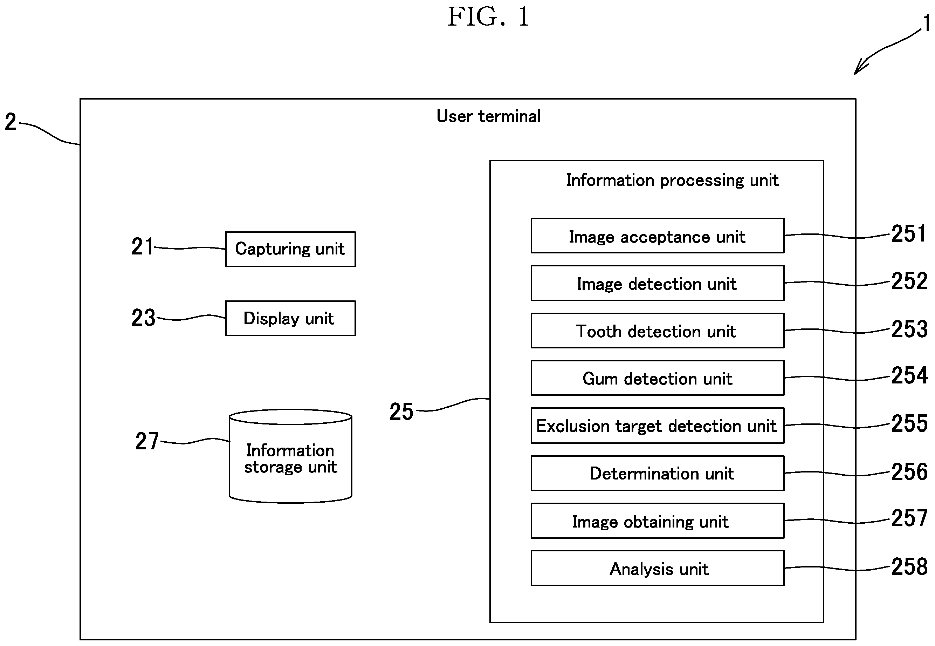

   FIG. 1 is a block diagram illustrating an oral cavity condition analysis system according to an embodiment of the present invention.
[FIG. 2]

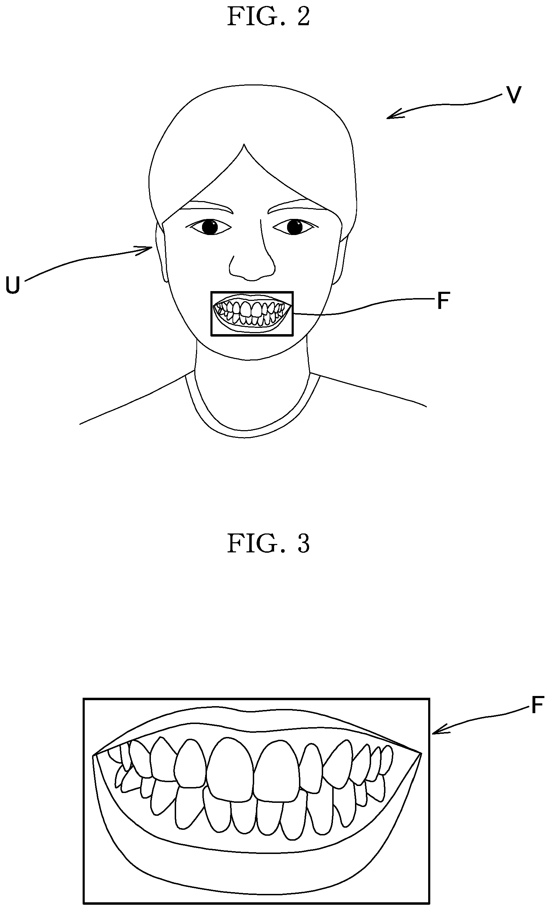

   FIG. 2 is a schematic diagram showing a face image captured by a capturing unit.
[FIG. 3]
   FIG. 3 is a schematic diagram showing a mouth image detected from the face image shown in FIG. 2.
[FIG. 4]

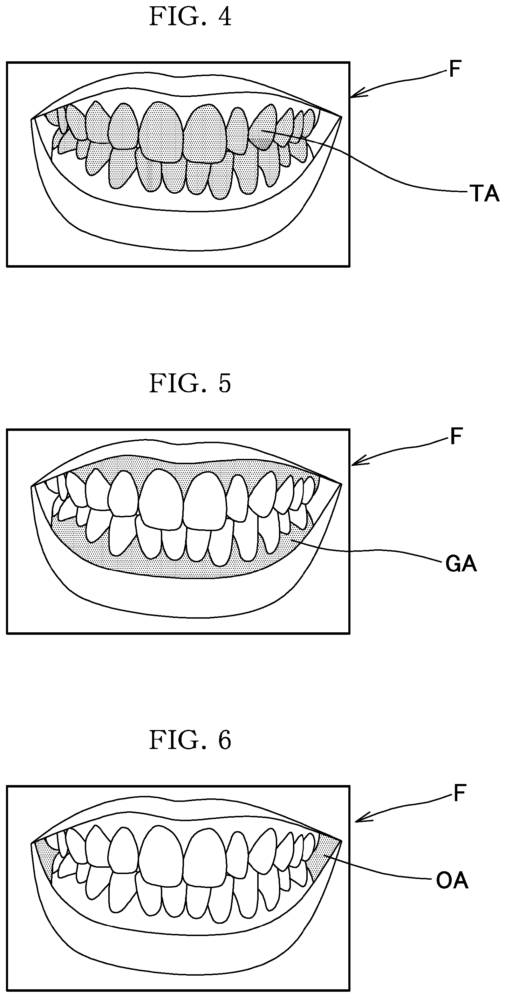

   FIG. 4 is a schematic diagram showing a tooth area detected by a tooth detection unit from the mouth image shown in FIG. 3.
[FIG. 5]
   FIG. 5 is a schematic diagram showing a gum area detected by a gum detection unit from the mouth image shown in FIG. 3.
[FIG. 6]
   FIG. 6 is a schematic diagram showing other areas detected by an exclusion target detection unit from the mouth image shown in FIG. 3.
[FIG. 7]

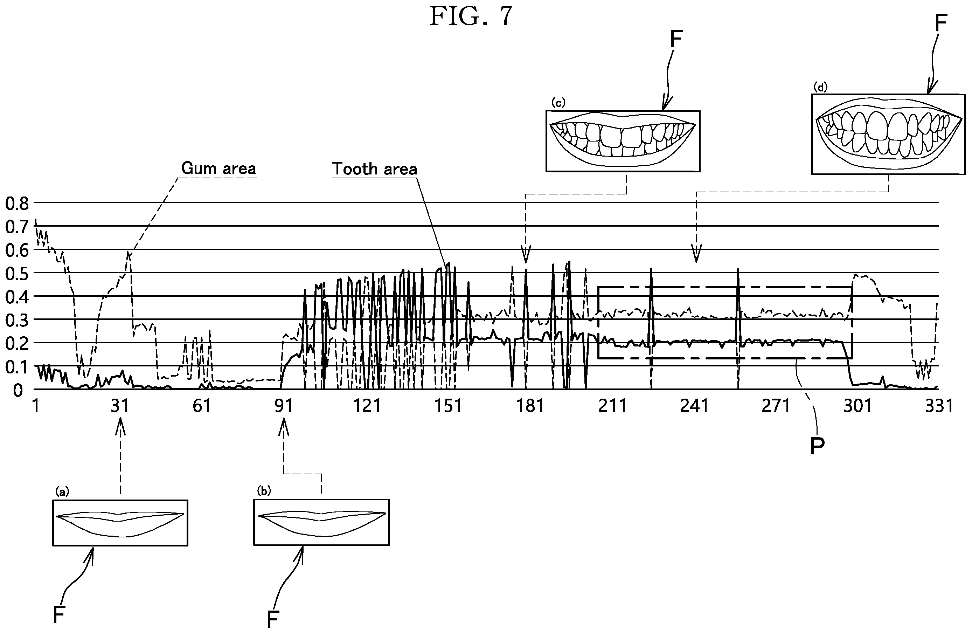

   FIG. 7 is a graph showing an example of time-series data for tooth area values and gum area values recorded by a determination unit at predetermined intervals.
[FIG. 8]

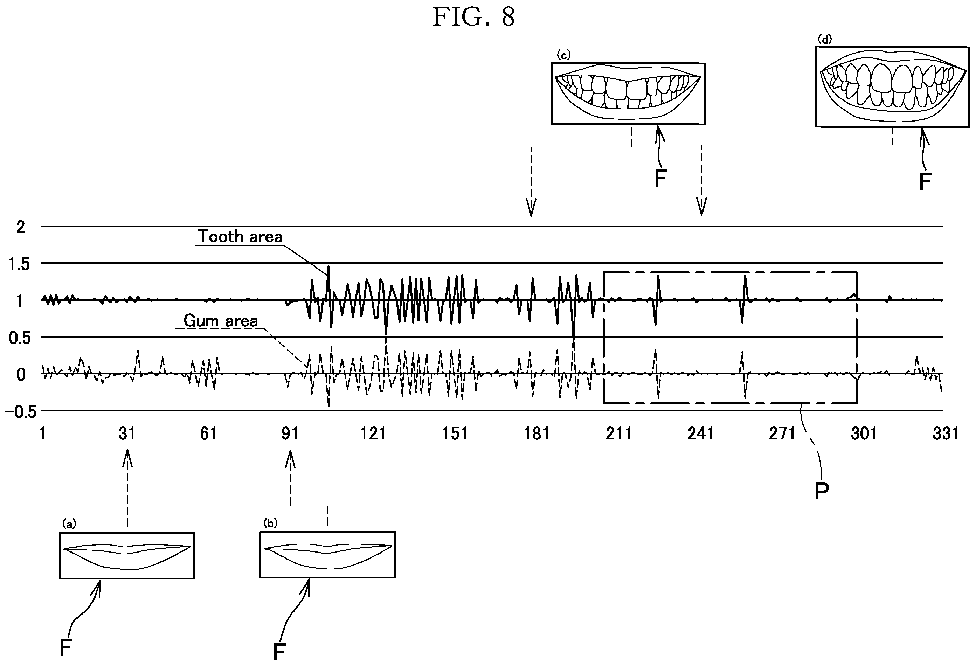

   FIG. 8 is a graph showing an example of a change amounts in tooth area TA values and gum area GA values in the time-series data shown in FIG. 7.
[FIG. 9]

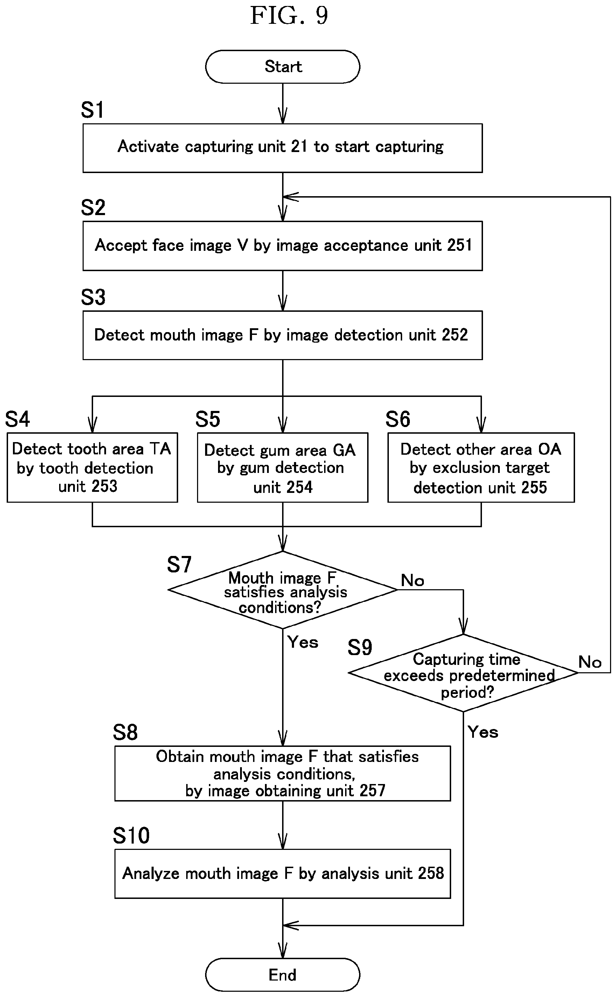

   FIG. 9 is a flowchart illustrating a workflow of the oral cavity condition analysis system shown in FIG. 1.
[FIG. 10]
   FIG. 10 includes schematic diagrams each illustrating a user operation in a conventional analysis system.

### DESCRIPTION OF EMBODIMENTS

Hereinafter, embodiments of the present invention will be described with reference to the drawings. As shown in FIG. 1, an oral cavity condition analysis system 1 is implemented on a user terminal 2 used by a user.

The user terminal 2 is provided at least with a capturing unit 21, a display unit 23, an information processing unit 25, and an information storage unit 27. The user terminal 2 may be a personal computer or a tablet device, as long as it is capable of accepting operations for analyzing oral cavity conditions, and a smartphone is preferable for its portability. In the following description, a smartphone will be used as the user terminal 2.

The capturing unit 21 is a camera that captures images of a user's face. The display unit 23 is a display screen, such as a touch panel, which accepts operations by a user and displays face images V captured continuously by the capturing unit 21. The capturing unit 21 is preferably located on a side of the display unit 23, which faces the user's face when the user operates the user terminal 2. The information processing unit 25 is a processing device such as a central processing unit (CPU). The information processing unit 25 performs operations and processing related to the oral cavity condition analysis system 1 in the user terminal 2. The information storage unit 27 is a storage device including memory such as RAM and ROM, as well as a hard disk drive. The information storage unit 27 stores various processing executed by the information processing unit 25 in the user terminal 2, information, and an oral cavity condition analysis program that causes the oral cavity condition analysis system 1 of the present invention to function. In addition, the information storage unit 27 stores at least one type of information used to analyze the oral cavity condition, such as a color or shape of the teeth and gums, skin of lips and skin around the lips (which are not teeth or gums), and shadows captured during capturing.

Next, the information processing unit 25 will be described. The information processing unit 25 executes the oral cavity condition analysis program stored in the information storage unit 27, at least causing an image acceptance unit 251, an image detection unit 252, a tooth detection unit 253, a gum detection unit 254, an exclusion target detection unit 255, a determination unit 256, an image obtaining unit 257, and an analysis unit 258 to operate.

First, when the user terminal 2 accepts a predetermined action from a user U via an application, for example, the capturing unit 21 starts capturing a face image V including multiple face images of the user U. The image acceptance unit 251 accepts the face image V as an input from the capturing unit. At this point, although the face image V accepted by the image acceptance unit 251 may be temporarily stored in the information storage unit 27 so as to be displayed on the display unit 23, the face image V is not stored as an image for analyzing the oral cavity condition. The image detection unit 252 detects a mouth image F, which includes a user's mouth and a surrounding area of the mouth as shown in FIG. 3, from the face image V including multiple face images as shown in FIG. 2. The image detection unit 252 can extract the mouth image F, which includes the user's mouth and the surrounding area of the mouth, from the face image V using object detection algorithms such as single shot multi-box detector (SSD) or semantic segmentation, for example.

As shown in FIG. 4, the tooth detection unit 253 detects a tooth area TA that indicates a location of teeth in the mouth image F. Specifically, the tooth detection unit 253 first converts an RGB color space of pixels contained in the mouth image F to an XYZ color space, and then converts the XYZ color space to a Lab color space. The tooth detection unit 253 extracts only a-dimensional pixels from the pixels that are contained in the mouth image F and have been converted to the Lab color space. The tooth detection unit 253 binarizes the a-dimensional pixels based on a predetermined threshold value and detects pixels labeled 1 as the tooth area TA.

As shown in FIG. 5, the gum detection unit 254 detects a gum area GA that indicates a location of gums in the mouth image F. Specifically, the gum detection unit 254 first applies a differential filter, such as a Sobel filter or a Prewitt filter, to each pixel that makes up the mouth image F. This emphasizes the details of edges and contours. The gum detection unit 254 binarizes the mouth image F, which is the result of application of the differential filter, based on a predetermined threshold value, and flattens the pixels by applying a flattening filter. The gum detection unit 254 forms a polygon surrounded by pixels labeled 0 on the outer side and pixels labeled 1 on the inner side, and detects the polygon as the gum area GA.

As shown in FIG. 6, the exclusion target detection unit 255 detects other areas OA that indicate areas in the oral cavity other than teeth and gums in the mouth image F. Specifically, the exclusion target detection unit 255 detects targets that could cause misrecognition, such as inner walls of the oral cavity or shadows in the oral cavity captured when the face image V is captured, and are excluded during the determination of the mouth image F performed by the determination unit 256 (described later) or during the analysis of the oral cavity condition performed by the analysis unit 258. The exclusion target detection unit 255 first converts the RGB color space of the pixels contained in the mouth image F to an HSV color space. The exclusion target detection unit 255 binarizes the pixels contained in the mouth image F based on a predetermined threshold value and detects pixels labeled 1 as the other area OA. Furthermore, the exclusion target detection unit 255 may also detect, as the targets for exclusion, lips and skin around the lips, which are included in the mouth image F as other area OA that indicates an area other than the teeth and gums in the oral cavity.

The determination unit 256 determines whether the mouth image F detected by the image detection unit 252 satisfies analysis conditions necessary for analyzing the oral cavity condition, based on the tooth area TA, the gum area GA, and the other area OA respectively detected by the tooth detection unit 253, the gum detection unit 254, and the exclusion target detection unit 255.

The image obtaining unit 257 obtains one or more mouth images F that the determination unit 256 has determined satisfying the analysis conditions, and stores the mouth images F as accumulated data in the information storage unit 27. The analysis unit 258 may use, for example, simple image analysis or statistical methods to analyze the oral cavity condition. Here, it is preferable to analyze the oral cavity condition using a machine learning model trained on the accumulated data in the information storage unit 27.

If a volume of the accumulated data for training the machine learning model is large, the accumulated data may be stored in an external storage device serving as a database, rather than being stored in the information storage unit 27 of the user terminal 2. Furthermore, the analysis of the oral cavity condition performed by the analysis unit 258 may be carried out by an external computing device. As another embodiment, the oral cavity condition analysis system 1 may be configured such that the user terminal 2 is electrically connected to a server via a network such as the Internet.

The oral cavity condition analysis system 1 according to the other embodiment executes the oral cavity condition analysis program on the server and carries out, on the server, processing corresponding to those performed by each of the image acceptance unit 251, image detection unit 252, tooth detection unit 253, gum detection unit 254, exclusion target detection unit 255, determination unit 256, image obtaining unit 257, and analysis unit 258.

Next, the determination unit 256 will be described. The determination unit 256 excludes the other area OA from a determination target in the mouth image F and determines whether the tooth area TA and the gum area GA in the mouth image F satisfy the analysis conditions. In other words, as shown in FIGS. 7 and 8 in which a value indicating a proportion of the tooth area TA detected by the tooth detection unit 253 in the mouth image F (hereinafter referred to as the tooth area TA value) and a value indicating a proportion of the gum area GA detected by the gum detection unit 254 in the mouth image F (hereinafter referred to as the gum area GA value) are recorded as the time-series data, the determination unit 256 determines whether the analysis condition is satisfied based on trends and changes in the tooth area TA value and the gum area GA value.

There are various analysis conditions required for analyzing the oral cavity condition. One or more conditions are included in the analysis conditions. At least one of the tooth area TA and the gum area GA in the mouth image F must satisfy the analysis conditions, and it is preferable that both the tooth area TA and the gum area GA satisfy the analysis conditions. Examples of the analysis conditions include: whether the tooth area TA in the mouth image F contains at least a specified number of teeth (crowns); and whether a dimension of the gum area GA in the mouth image F reaches a minimum dimension required for analyzing the oral cavity condition. Specific values and targets can be set according to the analysis method.

Specific examples of the determination performed by the determination unit 256 and the image obtaining performed by the image obtaining unit 257 are as follows. For the determination unit 256, the analysis conditions are set to: (1) both the tooth area TA value and the gum area GA value reach a predetermined threshold value (e.g., 0.15) or higher; (2) the state in which both values have reached the predetermined threshold value is maintained for a predetermined period (e.g., 30 frames) or longer; and (3) change amounts in the tooth area TA value of and the gum area GA value fall within a predetermined range (e.g., ±0.5). In addition, a predetermined determination period (e.g., 10 seconds or 300 frames) is set for the determination unit 256. The mouth image F used by the determination unit 256 for the determination is, for example, a mouth image F detected from a face image V captured at 30 fps.

Within the predetermined determination period, the determination unit 256 continuously repeats the determination as to whether the mouth image F satisfies the analysis conditions. The determinations by the determination unit 256 at points (a) through (d) during the predetermined determination period will be described.

The determination unit 256 determines whether the mouth image F at the time point (a) shown in FIGS. 7 and 8 satisfies the analysis conditions. Although the mouth image F at the time point (a) satisfies the analysis condition (3), it satisfies neither the analysis conditions (1) nor (2). For this reason, the mouth image F at the time point (a) is not obtained by the image obtaining unit 257.

The determination unit 256 determines whether the mouth image F at the time point (b) shown in FIGS. 7 and 8 satisfies the analysis conditions. Although the mouth image F at the time point (b) satisfies the analysis condition (3), like the mouth image F at the time point (a), it satisfies neither the analysis conditions (1) nor (2). For this reason, the mouth image F at the time point (b) is not obtained by the image obtaining unit 257.

The determination unit 256 determines whether the mouth image F at the time point (c) shown in FIGS. 7 and 8 satisfies the analysis conditions. The mouth image F at the time point (c) satisfies the analysis condition (3), like the mouth images F at the time points (a) and (b). Regarding the mouth image F at the time point (c), though the tooth area TA value exceeds the threshold value in terms of the analysis condition (1), the gum area GA value does not reach the threshold value in terms of the analysis condition (1). In addition, the mouth image F at the time point (c) does not satisfy the analysis condition (2). For this reason, the mouth image F at the time point (c) is not obtained by the image obtaining unit 257.

The determination unit 256 further determines whether the mouth image F at the time point (d) shown in FIGS. 7 and 8 satisfies the analysis conditions. The mouth image F at the time point (d) satisfies all of the analysis conditions (1) through (3). For this reason, the mouth image F at the time point (d) is obtained by the image obtaining unit 257.

In the determination performed by the determination unit 256, there may be more than one mouth image F that satisfies the analysis conditions (1) through (3). A bounding line P shown in FIGS. 7 and 8 encloses a period during which there are multiple mouth images F that satisfy the analysis conditions (1) through (3). Therefore, during the period enclosed by the bounding line P, which includes the time point (d), the image obtaining unit 257 obtains one or more mouth images F.

If, despite expiration of the determination period, the determination unit 256 determines that no mouth image F satisfies the analysis conditions (1) through (3), the determination unit 256 temporarily terminates the determination as to whether the mouth image F satisfies the analysis conditions.

Referring to FIG. 9, an operation flow of the oral cavity condition analysis system 1 according to the embodiment of the present invention will be described.

First, the capturing unit 21 is activated to start capturing (Step S1). For example, when a user operates an application on the user terminal 2, the application causes the capturing unit 21 in the user terminal 2 to start capturing. When the capturing unit 21 starts capturing images, the face image V including multiple face images of a user U is captured.

At this time, it is preferable that a guidance display unit (not shown) causes a guidance means to be displayed on the display unit 23 for guiding the user to a mouth-opening posture suitable for analyzing the oral cavity condition, namely, a posture in which teeth and gums are exposed. Although the guidance means is depicted as a horizontally elongated rectangular frame like the mouth, it may also be shaped in a frame of any other shape, including a frame shaped like teeth and gums. Furthermore, the guidance means may be a frame shaped like a face outline.

The image acceptance unit 251 accepts the face image V (Step S2). Although the face image V accepted by the image acceptance unit 251 may be temporarily stored in the information storage unit 27 so as to be displayed on the display unit 23, it is not stored as an image for analyzing the oral cavity condition.

The image detection unit 252 detects the mouth image F (Step S3). The image detection unit 252 detects the mouth image F by using a part of the face image V as a detection area. A detection area to be detected by the image detection unit 252 in the face image V displayed on the display unit 23 is indicated. At this time, a lower half of the vertically split face image V, specifically a mouth area, including the user's mouth and a surrounding area of the mouth, is designated as the detection area of the mouth image F, and the mouth image F is detected from the face image V.

In the mouth image F detected by the image detection unit 252, the tooth detection unit 253 detects the tooth area TA (Step S4), the gum detection unit 254 detects the gum area GA (Step S5), and the exclusion target detection unit 255 detects the other area OA (Step S6). It should be noted that in the flowchart shown in FIG. 9, steps S4, S5, and S6 are performed simultaneously, but they may also be performed in the order of steps S4, S5, and S6, or in any order.

The determination unit 256 determines whether the mouth image F satisfies the analysis conditions (Step S7). If the mouth image F satisfies the analysis conditions ("Yes" in Step S7), processing proceeds to Step S8. If the mouth image F does not satisfy the analysis conditions ("No" in Step S7), the processing proceeds to Step S9.

The image obtaining unit 257 obtains the mouth image F that satisfies the analysis conditions (Step S8). If the determination unit 256 determines that the mouth image F satisfies the analysis conditions ("Yes" in Step S7), the image obtaining unit 257 obtains one or more of the mouth images F that the determination unit 256 has determined satisfying the analysis conditions. The mouth image F obtained by the image obtaining unit 257 is temporarily stored in the information storage unit 27. At this point, it is preferable to employ a notification means, such as a flash effect that temporarily illuminates the display unit 23, to inform the user U that the capture of the mouth image F suitable for analyzing the oral cavity condition has been completed.

It is determined whether capturing time has exceeded a predetermined determination period (Step S9). If the determination unit 256 determines that the mouth image F does not satisfy the analysis conditions ("No" in Step S7), the determination unit 256 further determines whether a predetermined determination period during which whether the mouth image F satisfies the analysis conditions is determined has elapsed. If the predetermined determination period has not elapsed ("No" in Step S9), the processing returns to Step S2 and the processing is restarted from the acceptance of the face image V. If the predetermined determination period has elapsed ("Yes" in Step S9), the oral cavity condition analysis system 1 of the present invention terminates.

The analysis unit 258 analyzes the oral cavity condition (Step S10). The analysis unit 258 retrieves the mouth image F obtained by the image obtaining unit 257 from the information storage unit 27 and analyzes the oral cavity condition using, for example, a machine learning model trained on the accumulated data stored in the information storage unit 27. The analysis results processed by the analysis unit 258 are displayed on the display unit 23 of the user terminal 2, and the oral cavity condition analysis system 1 of the present invention is terminated. Furthermore, it is preferable to store the analysis results processed by the analysis unit 258 in the information storage unit 27 as new training data. In addition, the analysis unit 258 may employ other methods for analyzing the oral cavity condition, such as simple image analysis including binarization processing that binarizes the mouth image F, or a statistical method such as histogram analysis, for example.

Accordingly, the present invention provides an oral cavity condition analysis system and an oral cavity condition analysis program, which offer the convenience in automatically obtaining oral cavity images suitable for analyzing oral cavity conditions.

Although the embodiments of the present invention have been described, the present invention is not limited to these examples and may, of course, be practiced in various forms without departing from the scope of the invention.

### REFERENCE SIGNS LIST

- 1: Oral cavity condition analysis system
- 2: User terminal
- 21: Capturing unit
- 23: Display unit
- 25: Information processing unit
- 27: Information storage unit
- 251: Image acceptance unit
- 252: Image detection unit
- 253: Tooth detection unit
- 254: Gum detection unit
- 255: Exclusion target detection unit
- 256: Determination unit
- 257: Image obtaining unit
- 258: Analysis unit
- F: Mouth image
- GA: Gum area
- OA: Other area
- P: Bounding line
- TA: Tooth area
- U: User
- V: Face image

## Claims

1. An oral cavity condition analysis system comprising:
an image acceptance unit that continuously accepts a face image of a user as an input from a capturing unit, the face image having been captured by the capturing unit provided in a user terminal;
an image detection unit that detects an image containing a mouth area from the face image accepted by the image acceptance unit;
a tooth detection unit that detects a tooth area from the image containing the mouth area;
a gum detection unit that detects a gum area from the image containing the mouth area;
a determination unit that determines whether at least one of the tooth area and the gum area which are included in the image containing the mouth area satisfies a predetermined condition necessary for analyzing an oral cavity condition;
an image obtaining unit that obtains one or more images containing the mouth area, the images having been determined by the determination unit to satisfy the condition; and
an analysis unit that analyzes the oral cavity condition based on the image containing the mouth area, the image having been obtained by the image obtaining unit.

2. The oral cavity condition analysis system according to claim 1, comprising
an exclusion target detection unit that detects an exclusion target other than the tooth area and the gum area from the image containing the mouth area.

3. The oral cavity condition analysis system according to claim 1, wherein
the determination unit records at least one of the tooth area and the gum area as time-series data, and determines whether the image containing the mouth area satisfies the predetermined condition necessary for analyzing the oral cavity condition based on the time-series data, the tooth area and the gum area varying for each image containing the mouth area.

4. The oral cavity condition analysis system according to claim 3, wherein
the user terminal includes a display screen on which the face image is displayed, and the display screen is provided with a guidance display unit that displays a guidance means for allowing the mouth area to be guided to a predetermined position.

5. The oral cavity condition analysis system according to claim 3, wherein
the tooth detection unit extracts the tooth area from the image containing the mouth area using color space conversion and a predetermined threshold value, and
the gum detection unit applies a differential filter to the image containing the mouth area, and detects the gum area using a flattening filter and a predetermined threshold value.

6. An oral cavity condition analysis program that causes a computer to function as:
an image acceptance unit that continuously accepts a face image of a user as an input from a capturing unit, the face image being captured by the capturing unit provided in a user terminal;
an image detection unit that detects an image containing a mouth area from the face image accepted by the image acceptance unit;
a tooth detection unit that detects a tooth area from the image containing the mouth area;
a gum detection unit that detects a gum area from the image containing the mouth area;
a determination unit that determines whether at least one of the tooth area and the gum area which are included in the image containing the mouth area satisfies a predetermined condition necessary for analyzing an oral cavity condition;
an image obtaining unit that obtains one or more images containing the mouth area determined by the determination unit to satisfy the condition; and
an analysis unit that analyzes the oral cavity condition based on the image containing the mouth area, the image having been obtained by the image obtaining unit.

7. A computer readable recording medium that has an oral cavity condition analysis program recorded thereon for causing a computer to function as:
an image acceptance unit that continuously accepts a face image of a user as an input from a capturing unit, the face image being captured by the capturing unit provided in a user terminal;
an image detection unit that detects an image containing a mouth area from the face image accepted by the image acceptance unit;
a tooth detection unit that detects a tooth area from the image containing the mouth area;
a gum detection unit that detects a gum area from the image containing the mouth area;
a determination unit that determines whether at least one of the tooth area and the gum area which are included in the image containing the mouth area satisfies a predetermined condition necessary for analyzing an oral cavity condition;
an image obtaining unit that obtains one or more images containing the mouth area determined by the determination unit to satisfy the condition; and
an analysis unit that analyzes the oral cavity condition based on the image containing the mouth area, the image having been obtained by the image obtaining unit.

8. A method of analyzing an oral cavity condition, the method comprising:
continuously accepting a face image of a user as an input from a capturing unit, the face image having been captured by the capturing unit provided in a user terminal;
detecting an image containing a mouth area from the face image;
detecting a tooth area and a gum area from the image containing the mouth area;
determining whether at least one of the tooth area and the gum area which are included in the image containing the mouth area satisfies a predetermined condition necessary for analyzing an oral cavity condition;
obtaining one or more images containing the mouth area, the images having been determined to satisfy the condition; and
analyzing the oral cavity condition based on the image containing the mouth area, the image having been obtained.

9. The method of analyzing an oral cavity condition according to claim 8, further comprising
detecting an exclusion target other than the tooth area and the gum area from the image containing the mouth area.

10. The method of analyzing an oral cavity condition according to claim 8, further comprising:
recording at least one of the tooth area and the gum area as time-series data, the tooth area and the gum area varying for each image including the mouth area; and
determining whether the image containing the mouth area satisfies the predetermined condition necessary for analyzing the oral cavity condition based on the time-series data.
